# EUROPÄISCHE PATENTSCHRIFT

(11) **EP 1 057 470 B1**
(45) Veröffentlichungstag und Bekanntmachung des Hinweises auf die Patenterteilung: **26.09.2001**
(21) Anmeldenummer: 00108766.7
(22) Anmeldetag: 25.04.2000
(51) Int. Cl.: A61K 7/09, A61K 7/11, A61K 7/00

(54) **Aerosol-Schaumdauerwellzusammensetzung**
Aerosol foam composition for permanent waving
Composition d'aerosol mousseux pour l'ondulation permanente

(30) Priorität: 19.05.1999 DE 19922859
(43) Veröffentlichungstag der Anmeldung: 06.12.2000
(73) Patentinhaber: GOLDWELL GmbH, 64297 Darmstadt (DE)
(72) Erfinder: Bräutigam, Ina, 64285 Darmstadt (DE)

(56) Entgegenhaltungen:
- EP-A- 0 304 846
- EP-A- 0 307 086
- EP-A- 0 324 478
- EP-A- 0 815 827
- DE-A- 3 717 103
- DE-A- 19 904 014
- GB-A- 1 121 563
- US-A- 3 433 868
- SEKIYA, A., KUROSAWA, S.: "Preparation of ethane-series fluorohydrocarbons" STN INTERNATIONAL; FILE CAPLUS, AN1993:233460, 7. Juni 1993 (1993-06-07), XP002152225 & JP 04 364137 A 16. Dezember 1992 (1992-12-16)

## Beschreibung

Die vorliegende Erfindung betrifft ein Dauerwellmittel mit verbesserter Wellwirksamkeit.

Die Dauerwellung erfolgt bekanntlich in zwei Behandlungsschritten, der reduktiven Spaltung der Cystin-Disulfidbrücken des Haares durch Einwirkung eines Reduktionsmittels, und die anschließende Neutralisierung bzw. Fixierung durch Aufbringung eines Oxidationsmittels, wodurch die Cystin-Disulfidbrücken wiederhergestellt werden.

Das überwiegend eingesetzte Reduktionsmittel ist auch heute noch die Thioglykolsäure, z.B. in Form ihrer Salze, insbesondere des Ammoniumsalzes, obwohl zahlreiche andere Thioverbindungen für diesen Zweck vorgeschlagen wurden, die sich jedoch in der Praxis zumeist nicht durchgesetzt haben.

Die Thioglykolat enthaltenden Zusammensetzungen werden üblicherweise bei einem pH-Wert zwischen 7 und 10, insbesondere 8 und 9,5, eingesetzt

Es wurde auch schon vorgeschlagen, sogenannte Schaum-Dauerwellen anzuwenden, die den grundsätzlichen Vorteil aufweisen, sich beim Auftragen besser zu verteilen und deshalb auch eine gleichmäßigere Dauerwellung zu erzielen.

Da diese Dauerwellzusammensetzungen zweckmäßigerweise als Aerosole konfektioniert werden, wird auch eine vorzeitige Teiloxidation des Reduktionsmittels verhindert.

Derartige als Aerosole abgepackte und als Schaum abzugebende Dauerwellmittel sind beispielsweise in der DE 38 19 620 C2 und in der US-A 3 433 868 beschrieben.

Diese bekannten Zusammensetzungen, die als Treibmittel vorzugsweise Kohlenwasserstoffe wie Propan, n-Butan, Isobutan und deren Gemische oder Chlorfluorkohlenwasserstoffe enthalten, erzeugen aber einen relativ stabilen Schaum, was sowohl beim Friseur als auch bei der Kundin unerwünscht ist, da sich der Dauerwellvorgang dadurch nicht nur lange hinzieht, sondern die aufgebrachte Dauerwellflüssigkeit auch zum Abtropfen neigt, was natürlich absolut unerwünscht ist.

Die Erfindung geht daher von der Aufgabenstellung aus, eine Aerosol-Schaumdauerwell-zusammensetzung zu schaffen, die diese Nachteile nicht aufweist.

Diese wird durch eine Aerosol-Schaumdauerwellzusammensetzung gelöst, die, auf wäßriger Basis,
a) 2,5 bis 30 Gew.-% mindestens eines schwefelhaltigen Reduktionsmittels;
b) 0,25 bis 10 Gew.-% mindestens eines Tensids;
c) mindestens ein Alkalisierungsmittel zur Einstellung eines pH-Werts von 6,5 bis 10; und
d) 1 bis 15 Gew.-% 1,2-Difluorethan als alleiniges Treibmittel, jeweils berechnet auf die Gesamtzusammensetzung des Mittels, enthält.
Der optimale Druck liegt dabei zwischen etwa 3 und 5,5, vorzugsweise 4 und 5 bar.

Durch die Anwendung dieser Zusammensetzung wird ein gleichmäßiger Schaum erhalten, der nach etwa 7 bis 30 Sekunden zerfällt, wobei die Flüssigkeit in das Haar eindringt, damit eine vollständige Verformung gewährleistet ist und dem Anwender auch zeigt, welche Teile des gewickelten Haares nicht ausreichend imprägniert-wurden und deshalb noch einer Nachbehandlung bedürfen. Mit der erfindungsgemäßen Zusammensetzung wird also ein optimaler Mittelwert hinsichtlich Schaumstabilität und -qualität erreicht.

Die erfindungsgemäßen Dauerwellmittel enthalten eine reduzierende Schwefelverbindung. Bevorzugt sind Thioglykolsäure und Thiomilchsäure sowie deren Salze, insbesondere die Ammonium- und Ethanolaminsalze.

Weitere einsetzbare Thioverbindungen sind insbesondere Cystein bzw. dessen Hydrochlorid, Homocystein, Cysteamin, N-Acetylcystein, Thioglycerin, Ethandiolmonothioglykolat, 1,2-Propylenglykolmonothioglykolat (vgl. auch WO-A 93/1791), 1,3-Propandiol-monothioglykolat bzw. das daraus resultierende Isomerengemisch, 1,3-Butandiol- und 1,4-Butandiol-monothioglykolat bzw. deren Isomerengemische, Polyethylenglykol- wie Di-, Tri- und Tetraethylenglykolmonothioglykolate, Glycerinmonothiolactate und weitere Thiosäuren und deren Ester sowie Gemische derselben.

Auch die Verwendung anorganischer reduzierender Schwefelverbindungen wie Natrium-hydrogensulfit ist prinzipiell möglich.

Der Gesamtgehalt an Reduktionsmitteln in den erfindungsgemäßen Zusammensetzungen beträgt üblicherweise 2,5 bis etwa 30 Gew.-% absolut, und vorzugsweise 2,5 bis 15 Gew.-%, berechnet auf freie Thioglykolsäure als Bezugssubstanz.

Die Reduktionsmittel enthaltenden Dauerwellpräparate können, falls erforderlich, einen Gehalt an Alkalisierungsmitteln aufweisen. Die Menge ist abhängig vom reduzierenden Wirkstoff und dem angestrebten pH-Wert der Zusammensetzung. Vorzugsweise enthält die Reduktionsmittel-Zusammensetzung etwa 0,1 bis etwa 5, insbesondere etwa 0,5 bis etwa 2,5 Gew.-% desselben.

Bevorzugte Alkalisierungsmittel im Rahmen der Erfindung sind Ammoniumcarbamat, Ammoniak und/oder Ammonium(bi)carbonat. Es wird die Einstellung eines pH-Wertes im Bereich zwischen etwa 6,5 und etwa 10, vorzugsweise etwa 7 bis 9,5, angestrebt.

Die erfindungsgemäß zum Einsatz kommenden Aerosol-Schaumdauerwellmittel enthalten die Tenside in einer Menge von 0,25 bis 10, insbesondere etwa 0,5 bis 7,5, vor allem etwa 1 bis etwa 5 Gew.-%, berechnet auf die das Reduktionsmittel enthaltende Zusammensetzung.

Es handelt sich dabei vorzugsweise um die bekannten anionaktiven, nichtionischen und amphoteren bzw. zwitterionischen Produkte, die gegebenenfalls auch in Kombination untereinander zum Einsatz gelangen.

Geeignete anionaktive Tenside sind beispielsweise solche vom Sulfat-, Sulfonat-, Carboxylat- und Alkylphosphat-Typ, vor allem natürlich diejenigen, die üblicherweise zum Einsatz gelangen, beispielsweise die bekannten C₁₀-C₁₈-Alkylsulfate und insbesondere die entsprechenden Ethersulfate, beispielsweise C₁₂-C₁₄-Alkylethersulfat und Laurylethersulfat, insbesondere mit 1 bis 4 Ethylenoxidgruppen im Molekül, weiterhin Monoglycerid(ether)-sulfate, Fettsäureamidsulfate, die durch Ethoxylierung und anschließende Sulfatierung von Fettsäurealkanolamiden erhalten werden, und deren Alkalisalze sowie Salze langkettiger Mono- und Dialkylphosphate, die milde, hautverträgliche Detergentien darstellen, Acylisethionate, etc.

Es können auch Mischungen aus mehreren anionischen Tensiden eingesetzt werden, beispielsweise ein Gemisch aus einem α-Olefinsulfonat und einem Sulfosuccinat, vorzugsweise im Verhältnis 1:3 bis 3:1, oder einem Ethersulfat und einer Alkylamidoethercarbonsäure.

Eine weitere bevorzugte anionische Tensidgruppe, die in den erfindungsgemäßen Shampoos zusätzlich im Gemisch mit den erfindungsgemäß vorhandenen Tensiden zum Einsatz gelangt, sind C₈-C₂₂-Acylaminocarbonsäuren bzw. deren wasserlösliche Salze, vorzugsweise in einer Menge von 0,5 bis 10, insbesondere 1 bis 7,5 Gew.-%, berechnet auf die Gesamtzusammensetzung.

Besonders bevorzugt ist hierbei N-Lauroylglutamat, insbesondere als Natriumsalz. Weitere geeignete N-Acylaminocarbonsäuren sind beispielsweise N-Lauroylsarcosinat, N-C₁₂-C₁₈-Acylasparaginsäure, N-Myristylsarcosinat, N-Oleoylsarcosinat, N-Lauroylmethylalanin, N-Lauroyllysin und N-Lauroylaminopropylglycin, vorzugsweise in Form ihrer wasserlöslichen Alkali- oder Ammonium-, insbesondere Natriumsalze.

Weitere geeignete anionische Tenside sind C₈-C₂₀-Alkylpolyethercarbonsäuren bzw. deren Salze der Formel

R-(A)ₙ-O-CH₂COOX,

worin R eine C₈-C₂₀-Alkyl oder Alkenylgruppe, vorzugsweise eine C₁₂-C₁₄-Alkylgruppe, n eine Zahl A eine Ethylen- und/oder Propylenoxidgruppe, n eine Zahl von 1 bis 20, vorzugsweise 2 bis 17,und X H oder vorzugsweise ein Kation der Gruppe Natrium, Kalium, Magnesium und Ammonium, das gegebenenfalls hydroxyalkylsubstituiert sein kann, bedeuten.
Derartige Produkte sind seit längerem bekannt und im Handel, beispielsweise unter den Handelsnamen "AKYPO®" und "AKYPO-SOFT®".

Geeignete nichtionische Tenside sind Verbindungen aus der Klasse der Alkylpolyglucoside mit der allgemeinen Formel

R-O-(CH₂CH₂O)ₙ-Zₓ,

worin R eine Alkylgruppe mit 8 bis 20, vorzugsweise 10 bis 14 Kohlenstoffatomen, Zₓ einen Saccharidrest mit 5 bis 6 Kohlenstoffatomen, n eine Zahl von 0 bis 10, und x eine Zahl zwischen 1 und 5, vorzugsweise 1,1 bis 2,5 bedeuten.

Weitere geeignete nichtionische Tenside in den erfindungsgemäßen Mitteln sind C₁₀-C₂₂-Fettalkoholethoxylate.

Besonders geeignete C₁₀-C₂₂-Fettalkoholether sind die unter den Trivialnamen "Laureth", "Myristeth", Oleth", Ceteth", "Deceth", Steareth" und "Ceteareth" nach der CTFA-Nomenklatur mit Anfügung der Zahl der Ethylenoxid-Moleküle bezeichneten Alkylpolyglykolether, z.B. "Laureth-16":

Der durchschnittliche Ethoxylierungsgrad liegt dabei vorzugsweise zwischen etwa 5 und etwa 25, vorzugsweise etwa 10 und etwa 20.

Andere zusätzlich mitverwendbare nichtionische Tenside sind z.B. die verschiedenen Sor-bitanester, wie Polyethylenglykolsorbitanstearinsäureester, Fettsäurepolyglykolester oder auch Mischkondensate aus Ethylenoxid und Propylenoxid, wie sie beispielsweise unter der Handelsbezeichnung "Pluronics®" im Verkehr sind.

Weitere zusätzlich einsetzbare Tenside sind Aminoxide.
Solche Aminoxide gehören seit langem zum Stand der Technik, beispielsweise C₁₂-C₁₈-Alkyldimethylaminoxide wie Lauryldimethylaminoxid, C₁₂-C₁₈-Alkylamidopropyloder -ethylaminoxide, C₁₂-C₁₈-Alkyldi(hydroxyethyl)- oder -(hydroxypropyl)aminoxide, oder auch Aminoxide mit Ethylenoxid- und/oder Propylenoxidgruppen in der Alkylkette. Diese Aminoxide sind beispielsweise unter den Bezeichnungen "Ammonyx®", "Aromox®", oder "Genaminox®" im Handel.

Weitere fakultative Tensidbestandteile sind beispielsweise Fettsäuremono- und -dialkanol-amide, wie Cocosfettsäuremonoethanolamid und Myristinfettsäuremonoisopropanolamid, die auch als zusätzliche Schaumverstärker eingesetzt werden können.

Geeignete amphotere bzw. zwitterionische Tenside sind insbesondere die verschiedenen bekannten Betaine wie Fettsäureamidoalkylbetaine und Sulfobetaine, beispielsweise Laurylhydroxysulfobetain; auch langkettige Alkylaminosäuren wie Cocoaminoacetat, Cocoaminopropionat und Natriumcocoamphopropionat und -acetat haben sich als geeignet erwiesen.

In einzelnen können Betaine der Struktur 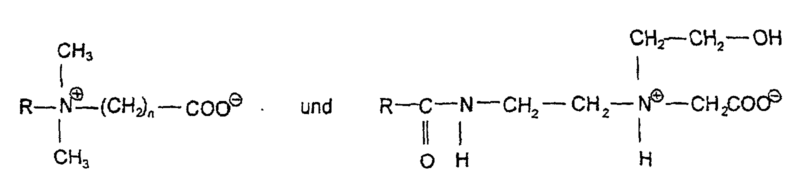 wobei R eine C₈-C₁₈-Alkylgruppe und n 1 bis 3 bedeuten,
Sulfobetaine der Struktur 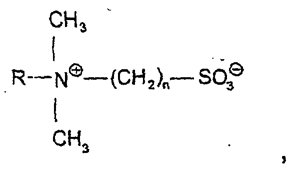 wobei R eine C₈-C₁₈-Alkylgruppe und n 1 bis 3 bedeuten, und Amidoalkylbeatine der Struktur 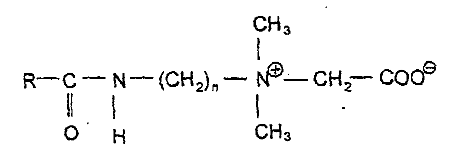 wobei R eine C₈-C₁₈-Alkylgruppe und n 1 bis 3 bedeuten.

Bevorzugt sind Fettsäureamidoalkylbetaine, insbesondere Cocoamidopropylbetain, und Cocoamphoacetat und-propionat, insbesondere deren Natriumsalze.
Besonders bevorzugt sind Gemische aus Cocoamidopropylbetain und Cocoamphoacetat, insbesondere im Gewichtsverhältnis 3:1 bis 1:3, vor allem 2:1 bis 1:1. Auch kationische Tenside können eingesetzt werden.
Geeignete kationische Tenside sind beispielsweise langkettige quaternäre Ammoniumverbindungen, die allein oder im Gemisch miteinander eingesetzt werden können, Cetyltrimethylammoniumchlorid, Dimethyldietylammoniumchlorid, Trimethylcetylammoniumbromid, Stearyltrimethylammoniumchlorid, Dimethylstearylbenzylammoniumchlorid, Benzyltetradecyldimethylammoniumchlorid, Dimethyl- dihydriertes-Talgammoniumchlorid, Laurylpyridiniumchlorid, Lauryldimethylbenzylammoniumchlorid, Behenyltrimethylammoniumchlorid, Lauryltrimethylammoniumchlorid, Tris-(oligooxyethyl)alkylammoniumphosphat, Cetylpyridiniumchlorid, etc. Gut geeignet sind auch die in der EP-A 472 107 geoffenbarten quaternären Ammoniumsalze.

Es können selbstverständlich auch Gemische aus den verschiedenen Tensiden, soweit sie miteinander kompatibel sind, verwendet werden.

Ein weiterer wünschenswerter Bestandteil der erfindungsgemäßen Reduktionsmittel-Zusammensetzungen ist ein C₃-C₆-Alkandiol bzw. dessen Ether, insbesondere Mono-C₁-C₃-alkylether.

Bevorzugte Substanzen sind in diesem Zusammenhang 1,2- und 1,3-Propandiol, 1-Methoxypropanol(-2), 1-Ethoxypropanol(-2), 1,3- und 1,4-Butandiol, Diethylenglykol und dessen Monomethyl- und Monoethylether sowie Dipropylenglykol und dessen Monomethyl- und Monoethylether.

Der Anteil dieser Diole liegt vorzugsweise zwischen 0,5 und 30, vorzugsweise etwa 1 bis etwa 15, insbesondere etwa 5 bis etwa 10 Gew.-% der Reduktionsmittel-Zusammensetzung.

Neben den C₃-C₆-Alkandiolen bzw. deren Ethern können zusätzlich auch Monoalkohole wie Ethanol, Propanol-1, Propanol-2 sowie Polyalkohole wie Glycerin und Hexantriol, Ethyl-carbitol, Benzylalkohol, Benzyloxyethanol sowie Propylencarbonat (4-methyl-1,3-dioxolan-2-on), N-Alkylpyrrolidone und Harnstoff Verwendung finden.

Weitere mögliche zusätzliche Bestandteile sind kationische, anionische, nichtionische und amphotere Polymere, vorzugsweise in einer Menge von etwa 0,1 bis etwa 5, insbesondere etwa 0,25 bis 2,5 Gew.-% der Gesamtzusammensetzung des Wellmittels,

Die erfindungsgemäßen Mittel können selbstverständlich alle in Dauerwellmitteln üblichen Stoffe enthalten, auf deren detaillierte Aufzählung hier verzichtet wird.

Zur Vermeidung von Wiederholungen wird hierzu auf den Stand der Technik verwiesen, wie er beispielsweise in "Ullmann's Encyclopedia of Industrial Chemistry", Vol. A12(1986), S. 588 bis 591, sowie insbesondere in der Monographie von K. Schrader, "Grundlagen und Rezepturen der Kosmetika", 2. Auflage (1989, Hüthig-Verlag), S. 823 bis 840, sowie in dem Übersichtsartikel von D. Hollenberg et al. In "Seifen-Öle-Fette-Wachse", 117 (1991), S. 81-87, beschrieben ist.

Die dort geoffenbarten Zusammensetzungen und Einzelbestandteile, auf die ausdrücklich Bezug genommen wird, können auch im Rahmen der vorliegenden Erfindung verwendet werden.

Die Abfüllung der erfindungsgemäßen Zusammensetzung mit dem 1,2-Difluorethan-Treib-mittel erfolgt auf übliche Weise, insbesondere in innenlackierte Weißblechdosen.

Falls erwünscht, kann vor dem Auftrag des Reduktionsmittels noch ein Vorbehandlungsmittel appliziert werden, wie es beispielsweise in der DE-A 37 40 926 beschrieben ist. Nach dem Aufbringen dieses Vorbehandlungsmittels wird das Haar aufgewickelt und die Reduktionsmittel-Zusammensetzung aufgetragen. Nach etwa 15- bis 30-minütiger Einwirkung und Spülung erfolgt die Fixierung mit üblichen, aus dem Stand der Technik hinreichend bekannten Peroxid- oder Bromat-Zusammensetzungen.

Ebenso kann selbstverständlich auch eine an sich bekannte Zwischenbehandlung zwischen Reduktions- und Neutralisierungsphase erfolgen.

Die folgenden Ausführungsbeispiele beschreiben die Erfindung weiter.

### Beispiel 1

| **Schaumdauerwelle für normales Haar** | |
|---|---|
| Natriumlaurylethersulfat | 3,00 (Gew.-%) |
| Ammoniumthioglykolat | 10,80 |
| Ammoniumhydrogencarbonat | 5,00 |
| Nichtionischer Emulgator (PEG-60 Hydriertes Ricinusöl) | 3,00 |
| Ethoxydiglykol | 1,50 |
| Chlorophyllin | 0,05 |
| Kationisches Polymer (Polyquaternium-11) | 0,50 |
| Parfum | 0,30 |
| Ammoniak | ad pH 8,6 |
| Wasser | ad 100,0 |

72 g dieser Zusammensetzung wurden mit 8 g 1,2-Difluorethan in eine innenlackierte Weißblechdose gefüllt.
Beim Ausbringen aus der Dose auf gewickeltes Haar wurde ein gleichmäßiger Schaum, der nach 15 bis 25 Sekunden zerfiel, erhalten.

Ein identisch zusammengesetztes, jedoch eine handelsübliche Propan/Butan-Treibmittelmischung nach dem Stand der Technik enthaltende Zusammensetzung ergab einen stabilen Schaum, der erst nach etwa 15 Minuten teilweise zerfiel.
Im Halbseitenversuch an einer Probandin zeigte sich nach der Fixierung mit einer handelsüblichen Wasserstoffperoxidzusammensetzung bei der mit der erfindungsgemäßen Aerosol-Schaumdauerwelle behandelten Hälfte ein deutlich ausgeprägteres, gleichmäßigeres Wellbild als bei der mit der Zusammensetzung nach dem Stand der Technik (Propan-Butan(Propan-Butan-Treibmittelgemisch) behandelten Hälfte.

In einem weiteren Vergleichsversuch wurde eine Haarhälfte mit der erfindungsgemäßen Zusammensetzung A, die andere mit einer ein Gemisch aus gleichen Teilen 1,2-Difluorethan und Monochlordifluorethan nach der US-A 3 433 868 anstelle des 1,2-Difluorethans enthaltenden, ansonsten identischen Zusammensetzung B behandelt.

Mit A wurde ein gleichmäßig verteilter, innerhalb von 15 bis 25 Sekunden zerfallender Schaum, der nach der Weiterbehandlung, Fixierung, Spülung und Trocknung zu einem ausgeprägten, gleichmäßigen Wellbild führte, erhalten.

Die Zusammensetzung B ergab einen grobporigen, wenig gleichmäßigen Schaum, der ohne zusätzliche manuelle Behandlung bis zu 10 Minuten sichtbar blieb. Die damit erhaltene Dauerwellung war weniger ausgeprägt und gleichmäßig,

## Patentansprüche

1. Aerosol-Schaumdauerwellzusammensetzung auf wäßriger Basis, enthaltend
a) 2,5 bis 30 Gew.-% mindestens eines schwefelhaltigen Reduktionsmittels;
b) 0,25 bis 10 Gew.-% mindestens eines Tensids,
c) mindestens ein Alkalisierungsmittel zur Einstellung eines pH-Wertes von 6,8 bis 10, und
d) 1 bis 15 Gew.-% 1,2-Difluorethan als alleiniges Treibmittel, jeweils berechnet auf die Gesamtzusammensetzung,

2. Zusammensetzung nach Anspruch 1, enthaltend 2 bis 10 Gew.-% 1,2-Difluorethan.

3. Verwendung von 1,2-Difluorethan als alleiniges Treibmittel in Aerosol-Schaumdauerwellzusammensetzungen auf wäßriger Basis, enthaltend
a) 2,5 bis 30 Gew.-% mindestens eines schwefelhaltigen Reduktionsmittels;
b) 0,25 bis 10 Gew.-% mindestens eines Tensids, und
c) mindestens ein Alkalisierungsmittel zur Einstellung eines pH-Wertes von 6,8 bis 10,
jeweils berechnet auf die Gesamtzusammensetzung,
zur Erzeugung eines feinporigen gleichmäßigen schnellbrechenden Schaums.

## Claims

1. Aerosol foam permanent waving composition on aqueous basis, comprising
a) 2.5 % to 30 % by weight of at least one reducing agent containing sulfur;
b) 0.25 % to 10 % by weight of at least one surfactant;
c) at least one alkalizing agent to adjust a pH-value of 6.8 to 10; and
d) 1 % to 15 % by weight of 1.2-difluoroethane as sole propellant, each calculated to the total composition,

2. Composition according to claim 1, comprising 2 to 10 % by weight of 1.2-difluoroethane.

3. Use of 1.2-difluoroethane as sole propellant in aerosol foam waving compositions on aqueous basis, comprising
a) 2.5 % to 30 % by weight of at least one reduction agent containing sulfur;
b) 0.25 % to 10 % by weight of at least one surfactant, and
c) at least one alkalizing agent to adjust a pH-value of 6.8 to 10, each calculated to the total composition,
for the achievement of a fine-porous, even, fast-breaking foam.

## Revendications

1. Composition de mousse/aérosol destinée aux permanentes, à base aqueuse, contenant
a) 2,5 à 30 % en poids d'au moins un agent réducteur contenant du soufre;
b) 0,25 à 10 % en poids d'au moins un tensioactif,
c) au moins un agent d'alcalinisation pour ajuster un pH de 6,8 à 10, et
d) 1 à 15 % en poids de 1,2-difluoroéthane en tant qu'agent propulseur uniqué, à chaque fois calculé par rapport au mélange total.

2. Composition selon la revendication 1, contenant 2 à 10 % en poids de 1,2-difluoroéthane

3. Utilisation du 1,2-difluoroéthane en tant que gaz propulseur unique dans des compositions de mousse/aérosol destinées aux permanentes, à base aqueuse, contenant
a) 2,5 à 30 % en poids d'au moins un agent réducteur contenant du soufre;
b) 0,25 à 10 % en poids d'au moins un tensioactif,
c) au moins un agent d'alcalinisation pour ajuster un pH de 6,8 à 10, et
d) 1 à 15 % en poids de 1,2-difluoroéthane en tant qu'agent propulseur unique, à chaque fois calculé par rapport au mélange total,
pour produire une mousse finement poreuse, homogène, à désémulsification rapide.
